# EUROPÄISCHE PATENTANMELDUNG

(11) **EP 1 043 000 A1**
(43) Veröffentlichungstag der Anmeldung: **11.10.2000**
(21) Anmeldenummer: 99810291.7
(22) Anmeldetag: 09.04.1999
(51) Int. Cl.: A61F 2/30

(54) **Positionierelement für einen Endoprothesenteil und Endoprothesenteil mit einem oder mehreren Positionierelementen**

(71) Anmelder: PLUS ENDOPROTHETIK AG, 6343 Rotkreuz (CH)
(72) Erfinder: Schmotzer, Hans, Dr., 5742 Kölliken (CH); Hässig, Christoph, 5024 Küttigen (CH); Learmonth Prof., Ian D., Bristol Royal Infirmary, Bristol BS2 8HW (GB)
(74) Vertreter: Patentanwälte Schaad, Balass, Menzl & Partner AG

(57) **Zusammenfassung**

Ein Positionierelement (1) für einen in einen Röhrenknochen implantierbaren Endoprothesenteil ist als ein ebenfalls in den Röhrenknochen implantierbarer Körper ausgebildet, der eine knochenseitige Aussenfläche (6) sowie einen inneren Aufnahmeraum (7) aufweist. Der Aufnahmeraum (7) ist zur Aufnahme des Endoprothesenteils vorgesehen. Der Körper weist zumindest in zwei sich bezüglich des in den Aufnahmeraum (7) eizusetzenden Endoprothesenteiles (20) gegenüberliegenden Bereichen unterschiedliche Wandstärken (W₂ ,W₃) auf. Das Positionierelement (1) ermöglicht eine optimale Plazierung des Endoprothesenteils im Markraum, wobei den anatomischen Besonderheiten des zu operierenden Röhrenknochens Rechnung getragen wird.

## Beschreibung

Die Erfindung betrifft ein Positionierelement für einen in einen Röhrenknochen implantierbaren Endoprothesenteil gemäss dem Oberbegriff des Anspruches 1 sowie einen Endoprothesenteil mit einem oder mehreren Positionierelementen gemäss dem Oberbegriff des Anspruches 2.

Ein Positionierelement für einen Endoprothesenteil ist beispielsweise aus der DE-A-196 13 200 bekannt. Es handelt sich um ein proximales Zentrierelement für einen Femur-Endoprothesenschaft in Form von einer Hülse, die in ein Implantantlager im Oberschenkelhals eingesetzt ist und im Innern passgerecht auf die Aussenfläche des aufzunehmenden Endoprothesenschaftes abgestimmt ist. Der Endoprothesenschaft wird durch die Hülse zentral in die Markraumhöhle eingeführt und dort zementfixiert. Es besteht die Gefahr, dass der Endoprothesenschaft zu weit posterior einzementiert wird, da der Femur im proximalen Bereich in anterior-posterioraler-Richtung eine Krümmung in Richtung zum Trochanter Minor aufweist. Der Zementmantel ist dann auf dieser Seite zu dünn, was in gewissen Fällen zu vorzeitigem Versagen des ganzen Systems führen kann.

Der vorliegenden Erfindung liegt die Aufgabe zugrunde, ein Positionierelement der eingangs genannten Art bzw. einen Endoprothesenteil mit einem oder mehreren Positionierelementen zu schaffen, mittels welchen mit Rücksicht auf die anatomischen Besonderheiten des zu operierenden Röhrenknochens eine optimale Plazierung des Endoprothesenteils im Markraum möglich ist.

Diese Aufgabe wird erfindungsgemäss durch ein Positionierelement mit den Merkmalen des Anspruches 1 sowie durch einen Endoprothesenteil mit den Merkmalen des Anspruches 2 gelöst.

Dadurch, dass der Endoprothesenteil im Markraum exzentrisch positioniert bzw. fixiert werden kann, kann dem Verlauf des Knochen-Hohlraumes besser Rechnung getragen werden.

Bevorzugte Weiterausgestaltungen des erfindungsgemässen Positionierelementes bilden den Gegenstand der abhängigen Ansprüche.

Die Erfindung wird im folgenden anhand der Zeichnung näher erläutert.

Es zeigen:
- Fig. 1: ein erstes Ausführungsbeispiel eines Positionierelementes für einen in einem Röhrenknochen implantierbaren Endoprothesenteil in perspektivischer Darstellung;
- Fig. 2: das Positionierelement nach Fig. 1 in Ansicht;
- Fig. 3: das Positionierelement nach Fig. 1 in Draufsicht;
- Fig. 4: einen Schnitt nach Linie IV-IV in Fig. 3;
- Fig. 5: einen Schnitt nach Linie V-V in Fig. 3;
- Fig. 6: im vertikalen, medial-lateralen Teilquerschnitt ein Femur mit einem eingesetzten Endoprothesenschaft und dem Positionierelement nach Fig. 1;
- Fig. 7: das Femur, den Endoprothesenschaft sowie das Positionierelement nach Fig. 6 im vertikalen, anterior-posterioralen Teilquerschnitt;
- Fig. 8: ein zweites Ausführungsbeispiel eines Positionierelementes in Ansicht;
- Fig. 9: das Positionierelement nach Fig. 8 in Draufsicht;
- Fig. 10: eine schematische Darstellung eines mit einem Positionierelement fest verbundenen Endoprothesenschaftes, medial-lateral angeordnet;
- Fig. 11: der Endoprothesenschaft nach Fig. 10 anterior-posterioral angeordnet;
- Fig. 12: eine weitere Variante eines mit einem Positionierelement verbundenen Endoprothesenschaftes in einer der Fig. 10 entsprechenden Darstellung;
- Fig. 13: der Endoprothesenschaft nach Fig. 12 anterior-posterioral angeordnet;
- Fig. 14: eine schematische Darstellung eines mit drei noppenartigen Positionierelementen versehenen Endoprothesenschaftes, medial-lateral angeordnet;
- Fig. 15: der Endoprothesenschaft nach Fig. 14 anterior-posterioral angeordnet;
- Fig. 16: eine schematische Darstellung eines mit zwei Positionierelementen in Form von Distanzstiften versehenen Endoprothesenschaftes, medial-lateral angeordnet; und
- Fig. 17: der Endoprothesenschaft nach Fig. 16 anterior-posterioral angeordnet.

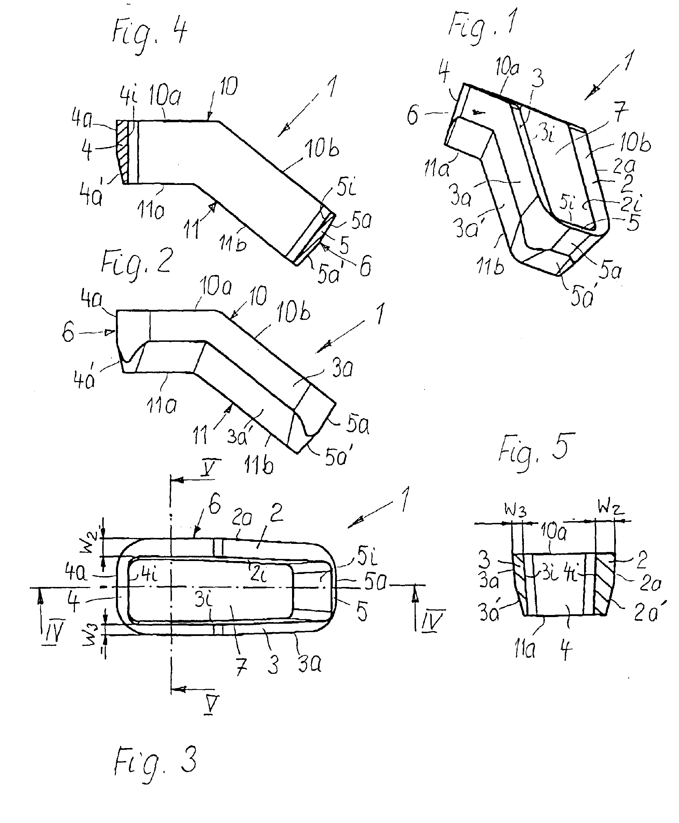

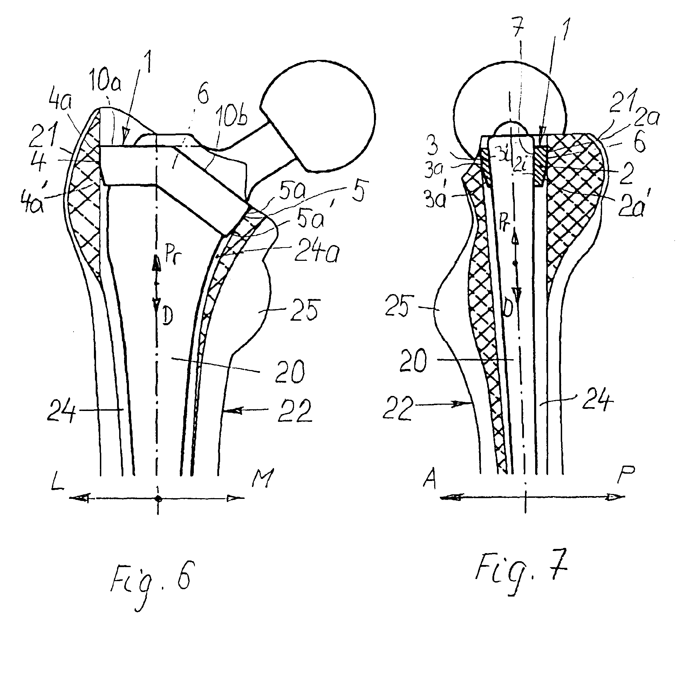

Gemäss Fig. 1 bis 5 ist ein Positionierelement 1 als ein hülsenförmiger Körper von einem im wesentlichen rechteckförmigen Querschnitt ausgestaltet, der zwei breitseitigen Wandteile 2, 3 sowie zwei schmalseitigen Wandteile 4, 5 aufweist. Die Aussenflächen der Wandteile 2, 3, 4, 5, die zusammen die Aussenfläche 6 des Positionierelementes 1 bilden, sind mit 2a, 3a, 4a, 5a bezeichnet. Die mit 2i, 3i, 4i, 5i bezeichneten Innenflächen der Wandteile 2, 3, 4, 5 umschliessen einen Innenraum 7.

Das Positionierelement 1 weist Stirnflächen 10, 11 auf, die jeweils durch zwei unter einem Winkel zueinander geneigte Teilflächen 10a, 10b bzw. 11a, 11b gebildet sind. Die im wesentlichen rechtwinklig zu den Teilflächen 10a, 11a bzw. 10b, 11b angeordneten Innenflächen 4i, 5i der schmalseitigen Wandteile 4, 5 schliessen zusammen ebenfalls einen Winkel, wobei sie in Richtung zur unteren Stirnfläche 11 hin konvergieren.

Die Innenflächen 2i, 3i der breitseitigen Wandteile 2, 3 sind im wesentlichen parallel zueinander angeordnet bzw. sie weisen einen nur leicht konvergierenden Verlauf in Richtung zur unteren Stirnfläche 11 hin auf.

Die Aussenflächen 2a, 3a, 4a, 5a aller Wandteile 2, 3, 4, 5 weisen jeweils einen Teilabschnitt 2a' bzw. 3a' bzw. 4a' bzw. 5a' auf, durch welchen die Wandteile in ihrer Wandstärke in Richtung zur unteren Stirnfläche 11 hin verjüngt sind.

Die breitseitigen Wandteile 2, 3 weisen unterschiedliche Wandstärken W₂ ,W₃ auf, so dass der Innenraum 7 gegenüber der Aussenfläche 6 des Positionierelementes 1 exzentrisch angeordnet ist.

Das vorstehend beschriebene Positionierelement 1 eignet sich in seiner Ausgestaltung insbesondere als ein proximales Positionierelement für die Implantation eines Endoprothesenschaftes in ein Femur, wie in Fig. 6 und 7 schematisch dargestellt. Dabei ist der Innenraum 7 des Positionierelementes 1 für die Aufnahme des in Fig. 6 und 7 mit 20 bezeichneten Endoprothesenschaftes vorgesehen und in seiner Form der Geometrie des Endoprothesenschaftes 20 angepasst. Die Aussenfläche 6 des Positionierelementes 1 entspricht in ihrer Geometrie einer Erweiterung 24a des Markraumes 24 im Femur- Schenkelhals 11, die im voraus mit einer Knochenraspel angefertigt wird. Das Vorformen des Markraumes, das Festlegen der Setztiefe für den Endoprothesenschaft, damit die Lage des Gelenkkopfes optimal ist, das Einfügen des Positionierelementes sowie des Endoprothesenschaftes und schliesslich das Einzementieren des Endoprothesenschaftes ist beispielsweise in der EP-Patentanmeldung Nr. 98810897.3 beschrieben und wird hier daher nicht näher erwähnt. Das Positionierelement 1 wird mit seiner Aussenfläche 6 in den resezierten Schenkelhals 21 des Femurs 22 eingedrückt und dabei auch der Endoprothesenschaft 20 in den Markraum 24 eingeführt. Der Innenraum 7 erstreckt sich dabei in proximal-distaler Richtung (Pfeil PrD in Fig. 6 und 7). Die breitseitigen Wandteile 2, 3 sind in lateral-medialer Richtung LM (Fig. 6) orientiert, und der die grössere Wandstärke W₂ aufweisende Wandteil 2 ist gemäss Fig. 7 in Richtung P (posterior), der die kleinere Wandstärke W₃ aufweisende Wandteil 3 in Richtung A (anterior) bezüglich der Femur-Achse angeordnet. Durch diese exzentrische Vorverlagerung des Innenraumes 7 und dadurch auch des Endoprothesenschaftes 20 im Markraum 24 in A-Richtung wird der Krümmung des Femurs 22 in Richtung zum Trochanter Minor (in Fig. 6 und 7 mit 25 bezeichnet) Rechnung getragen und vorgebeugt, dass der Endoprothesenschaft 20 nicht zu weit posterior implantiert wird.

Die sich in distaler Richtung D verjüngenden Teilabschnitte 2a', 3a', 4a', 5a' der Aussenflächen 2, 3, 4, 5 unterstützen das Einführen des Positionierelementes 1 in den Markraum 24. Beim in den Schenkelhals 21 eingedrückten Positionierelement 1 liegt vorzugsweise die Teilfläche 10b der oberen Stirnfläche 10 bündig zur Resektionsfläche des Schenkelhalses 21 (vgl. Fig. 6). Die Innenflächen 2i, 3i, 4i, 5i bilden Führungsflächen für den Endoprothesenschaft 20 beim Setzen desselben in proximal-distaler Richtung PrD.

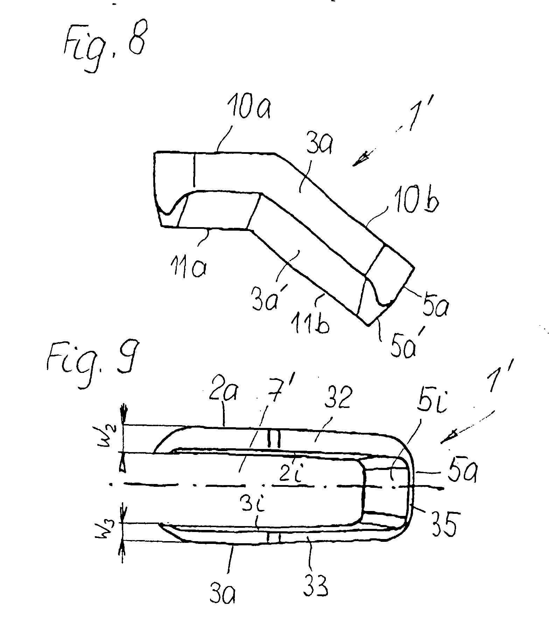

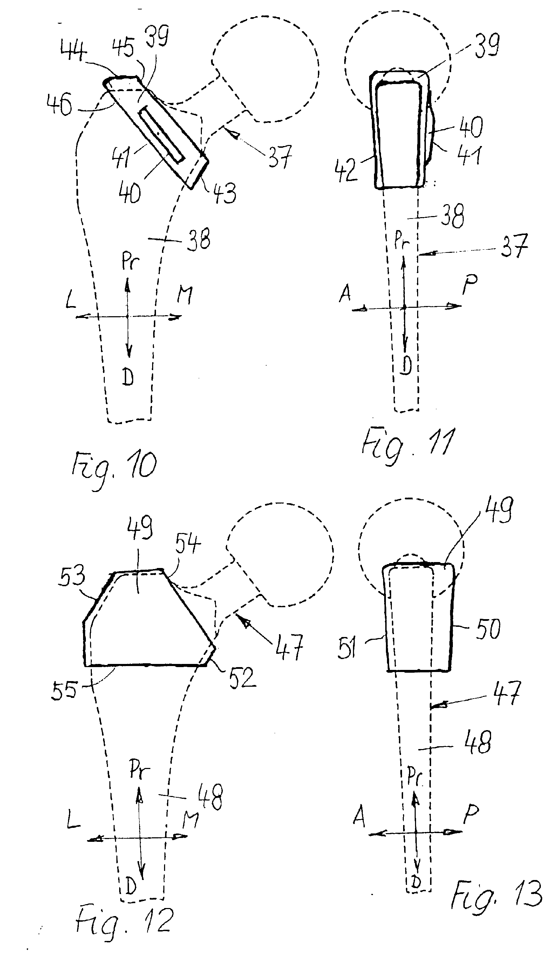

In Fig. 8 und 9 ist eine weitere Ausführungsform eines Positionierelementes 1' dargestellt, das als ein gabelförmiger Körper ausgestaltet ist und zwei Schenkel 32, 33 sowie einen Stammteil 35 aufweist. In ihrer Form entsprechen die Schenkel 32, 33 den breitseitigen Wandteilen 2, 3 des in Fig. 1 bis 7 dargestellten Positionierelementes 1 und der Stammteil 35 der schmalseitigen Wand 5. Einfachheitshalber sind die gleichbleibenden Aussenflächen, Innenflächen und Stirnflächen des Positionierelementes 1' mit den gleichen Bezugszeichen bezeichnet wie beim Positionierelement 1. Die die unterschiedlichen Wandstärken W₂ ,W₃ aufweisenden Schenkel 32, 33 und der Stammteil 35 begrenzen mit ihren Innenflächen 2i, 3i und 5i einen Innenraum 7' zur Aufnahme eines Endoprothesenteils. Das Positionierelement 1' kann ebenfalls insbesondere als proximales Zentrierelemnt für einen Femur-Endoprothesenschaft verwendet werden.

Beide Ausführungsformen des Positionierelementes 1 bzw. 1' sind für zementierte, flache Femur-Endoprothesenschäfte geeignet. Mit Vorteil können mehrere Positionierelemente mit unterschiedlicher Exzentrizität bzw. unterschiedlichem Wandstärkeverhältnis für die jeweiligen anatomischen Begebenheiten zur Verfügung stehen und wahlweise gewählt werden.

Das erfindungsgemässe Positionierelement könnte aber auch - selbstverständlich bei geeigneter Aussen- und Innenformgestaltung - bei anderen Endoprothesen wie Humerusnägeln, Tibianägeln etc. Anwendung finden, wobei die anatomischen Besonderheiten des jeweiligen Röhrenknochens berücksichtigt werden könnten.

Das erfindungsgemässe Positionierelement kann aus einem polymerisierten Knochenzement, beispielsweise aus Polymethylmetacrylat (PMMA), einem metallischen Werkstoff wie z.B. einer bikompatiblen Titanlegierung oder einem anderen Material hergestellt werden.

Statt Endoprothesenteile in einen Aufnahmeraum 7, 7' des Positionierelementes 1, 1' einzusetzen können aber auch ein oder mehrere Positionierelemente direkt am Endoprothesenteil angebracht und mit diesem kraft- und/oder formschlüssig verbunden sein. Derartige, mit einem oder mehreren Positionierelementen versehene Endoprothesenteile sind in Fig. 10 bis 17 dargestellt und werden im folgenden näher beschrieben.

In Fig. 10 und 11 ist eine Endoprothese 37 für ein Femur schematisch gestrichelt dargestellt, deren Endoprothesenschaft 38 kraft- und formschlüssig mit einem Positionierelement 39 verbunden ist. Das den Endoprothesenschaft 38 kragenförmig umschliessende Positionierelement 39 wird vorzugsweise durch Umspritzen des Endoprothesenschaftes 38 an seinem proximalen Ende hergestellt. Das Positionierelement 39 weist einen posterior angeordneten Vorsprung 40 auf, dessen im wesentlichen parallel zur Längsachse des Endoprothesenschaftes 38 verlaufende Aussenfläche 41 in einem grösseren Abstand von der Längsachse angeordnet ist, als eine gegenüberliegende, anterior-gerichtete Aussenfläche 42 des Positionierelementes 39 (Fig. 11). In medial-lateraler Richtung gesehen weist das Positionierelement 39 knochenseitige Aussenflächen 43, 44 auf. Beide gegebenfalls zueinander parallele Stirnflächen 45, 46 des Positionierelementes 39 sind zur Längsachse des Endoprothesenschaftes 38 geneigt. Der Vorsprung 40 erstreckt sich parallel zu den Stirnflächen 45, 46. Der mit dem Positionierelement 39 verbundene Endoprothesenschaft 38 wird wiederum im Markraum in A-Richtung vorverlagert plaziert, so dass der Krümmung des Femurs in Richtung zum Trochanter Minor hin Rechnung getragen wird.

In Fig. 12 und 13 ist eine Endoprothese 47 für ein Femur dargestellt, bei der das den Endoprothesenschaft 48 ebenfalls kragenförmig umschliessende Positionierelement 49 eine andere Form aufweist. Das Positionierelement 49 ist mit knochenseitigen Aussenflächen 50, 51, 52, 53 sowie Stirnflächen 54, 55 versehen. Die posterior-gerichtete Aussenfläche 50 liegt wiederum in einem grösseren Abstand von der Längsachse des Endoprothesenschaftes 48. Während die eine Stirnfläche 54 zur Längsachse geneigt ist, ist die andere Stirnfläche 55 im wesentlichen rechtwinklig zur derselben angeordnet. Das Positionierelement 49 ist wiederum mit dem Endoprothesenschaft 48 fest verbunden und wird vorzugsweise durch Umspritzen des Endoprothesenschaftes 48 hergestellt. Allerdings könnte auch ein loser, mit einem Aufnahmeraum für den Endoprothesenschaft versehener Positionierelement die gleich Aussenform wie das Positionierelement 49 aufweisen und beispielsweise im Spritzgussverfahren hergestellt werden.

Bei der in Fig. 14 und 15 dargestellten Variante einer Endoprothese 57 für ein Femur ist der Endoprothesenschaft 58 mit drei in proximal-distaler Richtung PrD gesehen in gleicher Höhe bzw. Ebene liegenden Positionierelementen 59 ausgestattet. Jedes Positionierelement 59 ist durch zwei sich in anterior-posterioraler Richtung AP gegenüberliegende, noppenartige Teilelemente 59a, 59p (Fig. 15) gebildet, die mit dem Endoprothesenschaft 58 fest verbunden sind. Sie können am Endoprothesenschaft 58 beispielsweise angespritzt, angeklebt oder in demselben eingepresst sein. Dabei ist der Abstand der durch das posterior angeordnete Teilelement 59p gebildeten Aussenfläche des Positionierelementes 59 von der Längsachse des Endoprothesenschaftes 58 grösser als derjenige der gegenüberliegenden, anterior angeordneten Aussenfläche des Teilelementes 59a. Selbstverständlich könnte die Endoprothese auch mit einer anderen Anzahl von aus gegenüberliegenden Teilelementen bestehenden Positionierelementen, die in proximal-distaler Richtung PrD nicht unbedingt in einer Ebene liegen müssen, ausgestattet sein. Die Teilelemente können beispielsweise auch eine Tropfenform aufweisen.

Zwei in PrD-Richtung unterschiedlich hoch liegende, noppenartige Teilelemente könnten auch beispielsweise den zur Längsachse des Endoprothesenschaftes 38 geneigten Vorsprung 40 am kragenförmigen Positionierelement 39 nach Fig. 10 ersetzen.

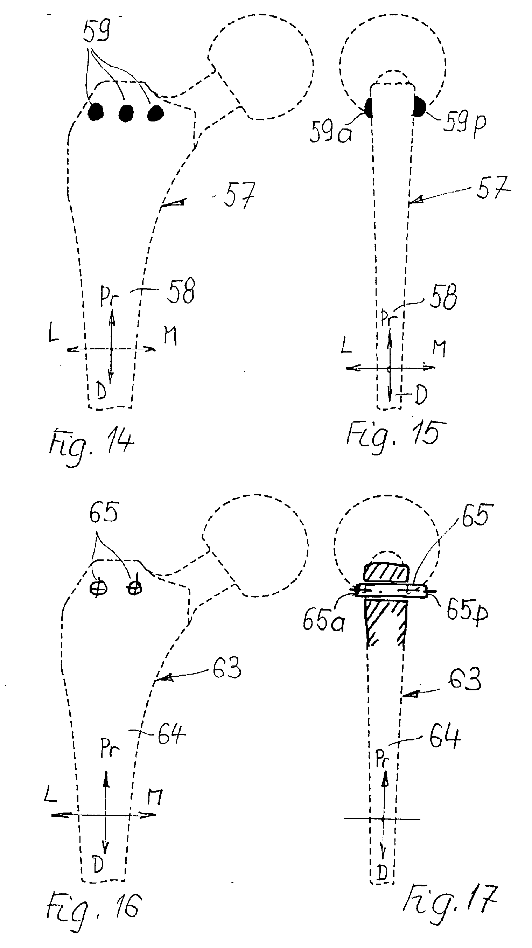

Bei der in Fig. 16 und 17 dargestellten Endoprothese 63 für ein Femur ist der Endoprothesenschaft 64 mit zwei Positionierelementen in Form von rechtwinklig zur seiner Längsachse angeordneten Distanzstiften 65 ausgestattet, die im Endoprothesenschaft 64 vorzugsweise eingepresst sind und diesen hindurchragen. Die Stirnflächen 65a, 65p dieser Distanzstifte 65 bilden die knochenseitigen Aussenflächen der Positionierelemente. Der Abstand der posterior angeordneten Stirnflächen 65p von der Längsachse des Endoprothesenschaftes 64 ist grösser als derjenige der anterior angeordneten Stirnflächen 65a. Die beiden Distanzstifte 65 liegen in proximal-distaler Richtung PrD gesehen in gleicher Höhe bzw. Ebene.

In Fig. 10 bis 17 wurden als Beispiel für mit erfindungsgemässen Positionierelementen fest verbundene Endoprothesenteile Femur-Endoprothesenschäfte dargestellt. Es könnten selbstverständlich auch andere Endoprothesenteile, z.B. Humerusnägel, Tibianägel und andere mit einem oder mehreren derartigen Positionierelementen fest verbunden sein.

Insbesondere die durch Umspritzen oder Anspritzen am Endoprothesenteil angebrachten Positionierelemente bestehen vorzugsweise aus Knochenzement, vor allem bei einzementierten Endoprothesen.

## Patentansprüche

1. Positionierelement für einen in einen Röhrenknochen (22) implantierbaren Endoprothesenteil (20), das als ein ebenfalls in den Röhrenknochen (22) implantierbarer Körper ausgebildet ist, der eine knochenseitige Aussenfläche (6) sowie einen inneren Aufnahmeraum (7) zur Aufnahme des Endoprothesenteils (20) aufweist, dadurch gekennzeichnet, dass zumindest in zwei sich bezüglich des in den Aufnahmeraum (7) einzusetzenden Endoprothesenteils (20) gegenüberliegenden Bereichen der Körper unterschiedliche Wandstärken (W₂ ,W₃) aufweist, um den Endoprothesenteil (20) exzentrisch im Markraum zu positionieren.

2. Endoprothesenteil für einen Röhrenknochen, der mit mindestens einem Positionierelement (39, 49, 59, 65) fest verbunden und mittels dessen knochenseitigen Aussenflächen im Markraum des Röhrenknochens fixierbar ist, dadurch gekennzeichnet, dass der Abstand von zumindest zwei sich bezüglich der Längsachse des Endoprothesenteils (38, 48, 58, 64) gegenüberliegenden Aussenflächen (41, 42; 50, 51; 59p, 59a; 65p, 65a) von der Längsachse unterschiedlich gross ist, um den Endoprothesenteil (38, 48, 58, 64) exzentrisch im Markraum zu positionieren.

3. Positionierelement nach Anspruch 1, dadurch gekennzeichnet, dass der Aufnahmeraum als ein von einer Innenfläche (2i, 3i, 4i, 5i) ganz oder teilweise umschlossener Innenraum (7, 7') ausgestaltet ist, wobei die Wand (2, 3, 4, 5) zwischen der Aussenfläche (6) und der Innenfläche (2i, 3i, 4i, 5i) zumindest in zwei sich bezüglich des Innenraumes (7) gegenüberliegenden Bereichen unterschiedliche Wandstärken (W₂ ,W₃) aufweist.

4. Positionierelement nach Anspruch 3, dadurch gekennzeichnet, dass der Innenraum (7) im wesentlichen rechteckförmig ausgestaltet und durch je zwei breitseitige Wandteile (2, 3) sowie zwei schmalseitige Wandteile (4, 5) umschlossen ist, wobei ein Paar der gegenüberliegenden Wandteile, vorzugsweise die breitseitigen Wandteile (2, 3) unterschiedliche Wandstärken (W₂,W₃) aufweisen.

5. Positionierelement nach Anspruch 4, dadurch gekennzeichnet, dass die breitseitigen Wandteile (2, 3) unterschiedliche Wandstärke (W₂ ,W₃) aufweisen und mit im wesentlichen parallel verlaufenden Innenflächen (2i, 3i) versehen sind.

6. Positionierelement nach Anspruch 5, dadurch gekennzeichnet, dass die Innenflächen (4i, 5i) der schmalseitigen Wandteile (4, 5) konvergierend verlaufen.

7. Positionierelement nach Anspruch 3, dadurch gekennzeichnet, dass der Körper gabelförmig ausgebildet ist, wobei die den Innenraum (7') mit ihren Innenflächen (2i, 3i, 5i) begrenzenden Wände bzw. Schenkel (32, 33) des gabelförmigen Körpers unterschiedliche Wandstärken (W₂,W₃) aufweisen.

8. Positionierelement nach Anspruch 1 oder einem der Ansprüche 3 bis 7, dadurch gekennzeichnet, dass die Aussenfläche (6) einen Teilabschnitt (2a', 3a', 4a', 5a') aufweist, durch welchen ein sich in seiner Aussenform verjüngender Bereich des Positionierelement-Körpers gebildet ist.

9. Positionierelement nach Anspruch 1 oder einem der Ansprüche 3 bis 8, dadurch gekennzeichnet, dass Stirnflächen (10, 11) des Körpers jeweils durch zwei unter einem Winkel zueinander geneigten Teilflächen (10a, 10b; 11a, 11b) gebildet sind.

10. Positionierelement nach Anspruch 1 oder einem der Ansprüche 3 bis 9, das als ein proximales Positionierelement für ein Femur-Endoprothesenschaft (20) ausgebildet ist, dadurch gekennzeichnet, dass der Körper dazu bestimmt ist, mit seiner Aussenfläche (6) in einen Markraum (24) des Femurs (22) eingeführt zu werden, derart, dass sich der Aufnahmeraum (7, 7') zur Aufnahme des Endoprothesenschaftes (20) in proxmial-distaler Richtung (PrD) erstreckt, die die unterschiedlichen Wandstärken (W₂ ,W₃) aufweisenden Wände bzw. Schenkel (2, 3; 32, 33) des Körpers in lateral-medialer Richtung (LM) orientiert sind und die die grössere Wandstärke (W₂) aufweisende Wand (2, 32) in anterior-posterioraler Richtung (AP) gesehen posterior gegenüber der Femur-Achse angeordnet ist.

11. Positionierelement nach Anspruch 10, dadurch gekennzeichnet, dass die Aussenflächen (2a, 3a, 4a, 5a) des Körpers je einen Teilabschnitt (2a', 3a', 4a', 5a') aufweisen, durch welche Teilabschnitte (2a', 3a', 4a', 5a') ein sich in seiner Aussenform verjüngender Bereich des Körpers gebildet ist und die dazu bestimmt sind, mit ihrem in distaler Richtung (D) konvergierendem Verlauf das Einführen des Körpers in den Markraum (24) zu erleichtern.

12. Endoprothesenteil nach Anspruch 2, dadurch gekennzeichnet, dass das Positionierelement (39, 49) den Endoprothesenteil (38, 48) zumindest teilweise kragenförmig umschliesst.

13. Endoprothesenteil nach Anspruch 12, dadurch gekennzeichnet, dass das Positionierelement in seiner Form dem Positionierelement (1, 1') nach Anspruch 1 oder einem der Ansprüche 3 bis 11 entspricht, jedoch mit dem Endoprothesenteil kraft- und/oder formschlüssig verbunden ist.

14. Endoprothesenteil nach Anspruch 2, dadurch gekennzeichnet, dass das Positionierelement durch einen im Endoprothesenteil (64) rechtwinklig zu seiner Längsachse angeordneten und diesen hindurchragenden Distanzstift (65) gebildet ist, dessen beide Stirnseiten (65a, 65p) die in unterschiedlichem Abstand zur Längsachse liegenden, knochenseitigen Aussenflächen bilden.

15. Endoprothesenteil nach Anspruch 2, dadurch gekennzeichnet, dass das Positionierelement (59) durch mindestens zwei am Endoprothesenteil (58) angebrachte, bezüglich der Längsachse des Endoprothesenteils (58) sich gegenüberliegende noppenartige Teilelemente (59a, 59p) gebildet ist, die mit den knochenseitigen Aussenflächen versehen und am Endoprothesenteil (58) vorzugsweise angespritzt, angeklebt oder im demselben eingepresst sind.

16. Endoprothesenteil nach Anspruch 14 oder Anspruch 15, dadurch gekennzeichnet, dass mehrere Positionierelemente (59, 65) am Endoprothesenteil in seiner proximal-distaler Längsrichtung gesehen auf gleicher Höhe bzw. in der gleichen Ebene angeordnet sind.
